# EUROPEAN PATENT APPLICATION

(11) **EP 1 464 309 A1**
(43) Date of publication of application: **06.10.2004**
(21) Application number: 03425147.0
(22) Date of filing: 07.03.2003
(51) Int. Cl.: A61F 7/03

(54) **Improved item of clothing having heating elements**

(71) Applicant: Time Out Italia S.r.l., 50132 Firenze (IT)
(72) Inventor: RAGONESI, Roberto, 50122 Firenze (IT)
(74) Representative: Modiano, Guido, Dr.-Ing.

(57) **Abstract**

An item of clothing (1), particularly for underwear, which has, on its inner surface (2), in contact with the skin, first temporary grip means (3) for second grip means. The second grip means are associated with at least one container (5) for one or more heating elements, such as elements that generate exothermic reactions.

## Description

The present invention relates to an improved item of clothing.

A problem that currently troubles many people, especially in the winter period or on particularly damp and cold days, is that they suffer from pains that are usually located at the shoulders, lower back, the sciatic nerve, and along the backbone.

These pains are usually caused by the presence of rheumatisms, or by past traumas or also by the increasingly widespread sedentary lifestyle of people.

Adverse weather conditions and sudden changes in humidity or temperature can increase the suffering of people due to the above-mentioned causes.

In order to alleviate these pains, it is known to perform therapies such as for example localized massages performed by specialized physiotherapists or to apply heat sources, such as heating pads, at the parts affected by the pain, so as to utilize the beneficial principles of heat therapy.

The main drawback of these known kinds of therapy is that they are particularly expensive, since they are usually performed by specialized personnel and produce substantial benefits only if they are extended over time.

Another drawback of these known kinds of therapy is the fact that they must be conducted in dedicated premises, with considerable difficulty for the patient to obtain prompt appointments.

Accordingly, often there is the problem due to the fact that while waiting for the therapies to start the pains persist and often worsen, with personal and work-related repercussions.

Another drawback consists of the fact that the treatments described above often require the patient to go to the dedicated premises, and this worsens the physical discomfort of the patient and causes a considerable reduction of spare time or working hours.

It is also known to use heating patches whose use, however, has many drawbacks: first of all, the patch must be positioned by another person for example in a chosen point of the back; moreover, the placement of the patch is not always correct, and it is therefore necessary to remove the patch several times, repositioning it in several points with the additional drawback that such patch loses its adhesiveness due to the fact that the skin usually releases substances that compromise adhesion to it.

The aim of the present invention is therefore to solve the above-mentioned problems, eliminating the drawbacks of the cited known art, by providing an item of clothing that allows the user to have immediately available a remedy against the onset of pains or rheumatisms that affect in particular the region of the trunk.

Within this aim, an object of the invention is to provide an item of clothing that allows the user to prepare simply and rapidly a specific remedy according to the particular region to be treated.

Another object is to provide an item of clothing that allows the user to treat himself in his own home, possibly while performing normal domestic, work-related or recreational activity.

Another object is to provide an item of clothing that can be used rapidly and without any risk or side effect for his health.

Another object is to provide an item of clothing that is structurally simple and has low manufacturing costs.

This aim and these and other objects that will become better apparent hereinafter are achieved by an item of clothing, characterized in that it has, on its inner surface, first temporary grip means for second grip means that are associated with at least one container for one or more heating elements.

Further characteristics and advantages of the invention will become better apparent from the detailed description of a particular but not exclusive embodiment thereof, illustrated by way of non-limitative example in the accompanying drawings, wherein:
Figures 1 and 2 are a side view and a rear view of the item of clothing according to the invention, worn by a female user;
Figure 3 is a perspective view of the container and of the heating element that can be accommodated in said container;
Figure 4 is a perspective view of the container of Figure 3.

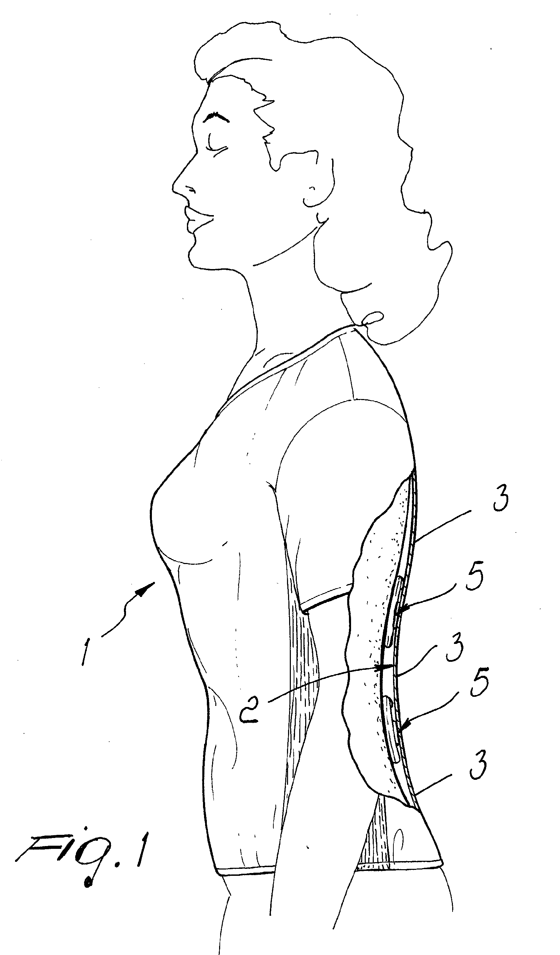

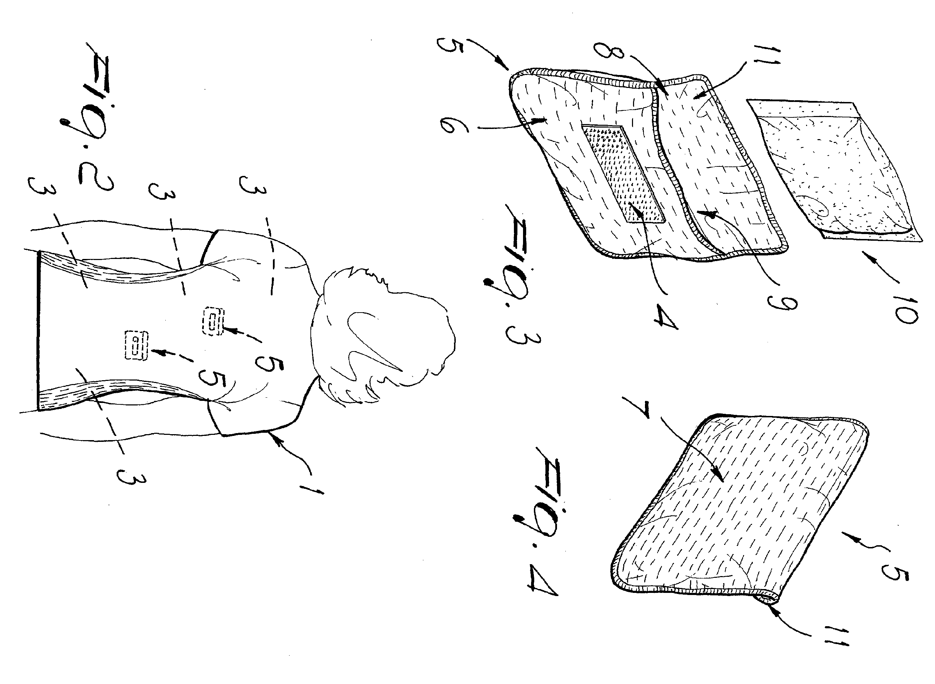

In the embodiments that follow, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

With reference to the figures, the reference numeral 1 designates an item of clothing, particularly an item of underwear such as an undershirt or a body suit with shorts, that can be worn under other items of clothing.

The undershirt 1 has an inner surface, designated by the reference numeral 2 and advantageously placed in contact with the skin, that has first means 3 for temporary grip for second grip means 4, which are in turn associated with at least one container 5.

In the illustrated embodiment, the first grip means 3 are constituted by the very fabric of which the undershirt 1 or at least its inner surface 2 is made.

For example, the inner surface 2 can be made of a preset fabric, whose characteristic is that it allows the temporary detachable grip of the container 5 or of a part thereof.

As an alternative, it is possible to provide inside the undershirt 1 one or more regions, not shown, that constitute the first grip means 3 for the second grip means 4.

In this particular embodiment, illustrated merely by way of example, the second grip means 4 are advantageously constituted by one or more strips of material such as the one known by the trademark Velcro, and are associated with the container 5 beforehand.

The container 5 can be constituted for example by a bag or pouch, which is advantageously rectangular and has a first front face 6, a second rear face 7, and an opening 8 obtained for example in an upper region for accessing an internal cavity or pocket 9.

The detachable and selectively arrangeable engagement of the container 5 with the inner surface 2 of the undershirt 1 is obtained by means of the temporary interconnection of the second grip means 4 with the first grip means 3.

The second grip means 4 are associated with the first face 6 or preferably with the second face 7 of the container 5.

In the pocket 9 of the container 5 it is possible to accommodate advantageously at least one heating element 10, such as an enclosure that accommodates substances that generate exothermic chemical reactions upon contact with air.

Once the heating element 10 has been placed in the container 5, it is possible to close the opening 8, for example by means of a flap 11 that protrudes upward from the upper face 7 and can be tilted in front of the front face 6.

It is possible to provide means for temporarily mutually fastening the flap 11 and the front face 6 of the container 5, such as for example a zip fastener, a button, a lace or two strips of material known by the trademark Velcro.

Operation is therefore as follows: with reference to the figures, once the painful areas have been located, the undershirt 1 is put on after positioning one or more containers 5 on each region to be treated.

Each container 5 accommodates one or more heating elements 10, which are activated beforehand by extracting them from a sealed bag, in order to achieve the contact of the internal chemical substances with the air.

The presence of the containers, which radiate heat, at the regions to be treated allows very easy and comfortable use of heat therapy.

To increase the effectiveness of the heating, it is possible to provide on the undershirt one or more elasticized bands that are suitable to increase the tightness of the fit of the inner surface 2 of the item of clothing; said one or more bands can be obtained along an axis that is longitudinal or transverse or oblique with respect to the item of clothing, or the improved item of clothing can be made entirely of elasticized fabric.

It has thus been found that the invention has achieved the intended aim and objects, an invention having been provided which allows the user to have a remedy that is effective against the onset of pains or rheumatisms that affect one or more regions of the trunk.

The item of clothing described above in fact allows the user to prepare simply and rapidly a specific remedy depending on the particular region to be treated and to perform simple adjustments of the position of the containers where necessary.

This is a considerable advantage of the present invention over known therapeutic/heating patches; by way of the present invention, one can position the container 5 with the heating element 10 several times and in different regions until the most precise and effective region, in which the heating element is located precisely on the painful spot, is found.

These different placements in different regions of the body can be performed by the same person that requires the treatment and are not possible when using said known adhesive heating patches, which after their first bonding, if removed to be positioned more precisely, are unable to reattach.

Another important advantage of the item of clothing according to the present invention is the fact that it allows partial use of the heating elements and therefore a cost saving for example with respect to the use of said known types of patch, which cannot be used partially.

Partial use of the item of clothing according to the present invention is in fact possible simply by inserting the heating element, after a few hours of use, in a hermetic bag, which can be associated for example with the item of underwear: the absence of air in the bag blocks the exothermic reaction, which resumes when said bag is reopened, in order to allow to reposition the heating element 10 in the container 5 and therefore in contact with the aching part.

The item of clothing according to the invention can be used directly in one's own home even while performing everyday activities, without any risk or side effect for the health of the wearer of the item of clothing.

The invention also allows, by way of the possibility to obtain heat that is localized on the suffering part, a quicker and optimum recovery after a muscle injury affecting the back, even for individuals performing a sports activity: the item of underwear can in fact be used even during the first sessions of rehabilitation exercise in which the suffering part is protected and aided by the application of heat.

The invention is of course susceptible of numerous modifications and variations, all of which are within the scope of the appended claims.

Thus, for example, it is possible to provide items of clothing that are different from the one described, to be used in contact with the skin or to be worn over underwear, so as to allow the user to position the heated enclosures in various regions of the human body.

The materials used, as well as the dimensions that constitute the individual components of the invention, may of course be more pertinent according to specific requirements.

The various means for performing certain different functions need not certainly coexist only in the illustrated embodiment but can be present per se in many embodiments, including ones that are not illustrated.

The characteristics that are indicated as advantageous, convenient or the like may also be omitted or replaced with equivalents.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An item of clothing, **characterized in that** it has, on its inner surface, first temporary grip means for second grip means that are associated with at least one container for one or more heating elements.

2. The item of clothing according to claim 1, **characterized in that** it is constituted by an item of underwear in which said inner surface placed in contact with the skin has one or more regions that comprise said first temporary grip means for said second grip means.

3. The item of clothing according to claims 1 and 2, **characterized in that** said first grip means are constituted by the very fabric of which at least one part of said inner surface of said item of clothing is made.

4. The item of clothing according to one or more of the preceding claims, **characterized in that** said inner surface is at least partially made of a preset fabric that is suitable to allow the temporary detachable grip of at least one part of said container.

5. The item of clothing according to one or more of the preceding claims, **characterized in that** said container is made of soft material, such as a fabric, in order to form a bag-like or pouch-like shape.

6. The item of clothing according to claims 1 and 5, **characterized in that** said container has a substantially polygonal shape which includes a first front face, a second rear face, and an opening, provided approximately in an upper region, for accessing an internal cavity or pocket.

7. The item of clothing according to one or more of the preceding claims, **characterized in that** said second grip means, constituted by one or more strips of a material such as the one known by the trademark Velcro, are associated beforehand with the rear face of said container.

8. The item of clothing according to claims 1 and 7, **characterized in that** said second grip means allow the detachable and selectively arrangeable engagement of said container with said first means for gripping the internal surface of said item of clothing.

9. The item of clothing according to one or more of the preceding claims, **characterized in that** said one or more heating elements can be accommodated temporarily in said internal cavity or pocket of said container.

10. The item of clothing according to claims 1 and 9, **characterized in that** said internal cavity or pocket can be closed temporarily by arranging a flap, which protrudes upward with respect to said second rear face of said container, in front of said first front face.

11. The item of clothing according to claims 1 and 10, **characterized in that** it has third means for temporarily mutually fastening said flap and said first front face of said container, such as a zip fastener, a button, a lace or a pair of strips of material known by the trademark Velcro.

12. The item of clothing according to one or more of the preceding claims, **characterized in that** each one of said heating elements comprises an enclosure that contains substances that generate exothermic chemical reactions upon contact with air.
